(19) Europäisches Patentamt

European Patent Office

Office européen des brevets

(11) EP 0 641 178 B1

(12) EUROPÄISCHE PATENTSCHRIFT

(45) Veröffentlichungstag und Bekanntmachung des Hinweises auf die Patenterteilung:
05.02.1997 Patentblatt 1997/06

(21) Anmeldenummer: 93909763.0

(22) Anmeldetag: 13.05.1993

(51) Int. Cl.⁶: $A61B\ 17/04$, $A61B\ 17/28$

(86) Internationale Anmeldenummer:
PCT/DE93/00418

(87) Internationale Veröffentlichungsnummer:
WO 93/24060 (09.12.1993 Gazette 1993/29)

(54) **CHIRURGISCHES NÄHINSTRUMENT**

SURGICAL SUTURE INSTRUMENT

INSTRUMENT CHIRURGICAL DE SUTURE

(84) Benannte Vertragsstaaten:
AT BE CH DE FR GB LI NL

(30) Priorität: 23.05.1992 DE 4217202

(43) Veröffentlichungstag der Anmeldung:
08.03.1995 Patentblatt 1995/10

(73) Patentinhaber: Forschungszentrum Karlsruhe GmbH
76133 Karlsruhe (DE)

(72) Erfinder:
• SCHÜLKEN, Heinrich
D-7513 Stutensee-Staffort (DE)
• NEEB, Roland
D-6833 Waghäusel 2 (DE)
• SCHLIPF, Karl
D-7500 Karlsruhe 1 (DE)
• KUNTZ, Bertram
D-6749 Steinfeld (DE)

(74) Vertreter: Rückert, Friedrich, Dr.
Forschungszentrum Karlsruhe GmbH
Patente und Lizenzen
Weberstrasse 5
76133 Karlsruhe (DE)

(56) Entgegenhaltungen:
EP-A- 0 121 362          WO-A-93/01750
DE-A- 3 716 764          GB-A- 2 157 180
US-A- 1 449 087          US-A- 4 078 305
US-A- 4 236 470          US-A- 4 306 560
US-A- 4 614 187          US-A- 4 655 223
US-A- 4 935 027

## Beschreibung

Die Erfindung betrifft ein chirurgisches Nähinstrument gemäß dem Oberbegriff des ersten Patentanspruchs.

Ein solches chirurgisches Nähinstrument ist aus der US 4,935,027 (Yoon) bekannt. Dieses Nähinstrument ist sowohl für die konventionelle als auch für die minimalinvasive (MIC) Chirurgie geeignet. Es besteht aus einem äußeren und einem inneren Rohr, die gegeneinander axial verschiebbar sind, aus deren distalen (dem Operateur abgewandten, in den Körper eines Patienten einzuführenden) Enden zwei zangen- oder pinzettenartige Backen oder Haken hervorragen, die hohl sind und in deren Hohlraum das Nähgut geführt wird. Aus der Druckschrift geht hervor, daß beide Backen oder Haken beweglich sein können oder die eine Backe feststeht und nur die andere beweglich ist. Am proximalen (dem Operateur zugewandten) Ende befindet sich ein Griffstück, das teilweise mit dem äußeren Rohr und teilweise mit dem inneren Rohr fest verbunden ist. Über das Griffstück werden die Backen oder Haken bewegt. Innerhalb des inneren Rohrs befindet sich die Zuführung für das Nähgut. Mit diesem Nähinstrument können lediglich Einzelknopfnähte angelegt werden, wobei jede Naht mit einem auf das Gewebe hin verschiebbaren Knoten gesichert werden muß. Die Anlage der Knoten erscheint kompliziert und zeitaufwendig. Ferner wird das Gewebe nicht nur vom Nähgut, sondern auch von den Haken durchstochen.

Aus der DE 31 41 647 A1 ist ein chirurgisches Instrument zum Anlegen von Klammern bekannt. Dieses Instrument enthält einen langgestreckten Körper mit einem Handgriff am proximalen Ende. Am distalen Ende des Körpers ist eine feststehende Stützbacke mit einer Matrize angeordnet. In der Matrize ist eine Aushöhlung zum Klammerschluß vorgesehen. Weiterhin ist am distalen Ende des Körpers eine bewegliche Andrückbacke mit einem Klammeraufschieber angeordnet. Die Andrückbacke ist am Körper in Richtung der genannten Matrize hin- und hergehend bewegbar befestigt. Ferner weist das Instrument einen Antrieb zum Verstellen der Andrückbacke und des Klammeraufschiebers auf. Die bewegliche Andrückbacke enthält einen Kanal für die Zuführung des Drahts. An der Andrückbacke ist ein Messer in zum Austrittsende des Kanals rechtwinkliger Richtung hin- und hergehend bewegbar angeordnet. Das Messer dient zum Abtrennen von Drahtstücken und ist mit dem obengenannten Antrieb kinematisch verbunden.

Das bekannte Instrument ist für die minimalinvasive Chirurgie wegen seiner Konstruktion und Größe nicht geeignet.

Ein weiteres Nähinstrument ist aus der EP 0 174 843 A2 bekannt. Dieses Nähinstrument besteht aus einem Block, der einen einseitig offenen Hohlraum umschließt. In den Hohlraum ist eine Nähgut-Haltevorrichtung integriert, die über ein Griffstück am proximalen Ende betätigt werden kann. In den Hohlraum mündet ferner ein Kanal, der eine Nadel mit einem Öhr aufnimmt, wobei das Nähgut durch das Öhr läuft. Der Kanal liegt der Nähgut-Haltevorrichtung gegenüber. Weiterhin mündet in den Hohlraum eine Saugvorrichtung. Der Block wird so auf das zu vernähende Gewebe aufgesetzt, daß der Hohlraum durch das Gewebe abgeschlossen wird. Anschließend wird der Hohlraum über die Saugvorrichtung evakuiert, so daß das Gewebe in einer doppelten Lage in den Hohlraum eintritt. Danach wird mit Hilfe der Nadel und der Nähgut-Haltevorrichtung eine Naht angelegt und schließlich die Saugvorrichtung belüftet, so daß das Gewebe die ursprüngliche Lage wieder einnimmt. Das Nähinstrument ist nur zum Übernähen von geschlossenem Gewebe geeignet, da es nur in diesem Fall mit Hilfe der Saugvorrichtung in den Hohlraum gebracht werden kann. Eine Versorgung von Wundrändern ist nicht möglich.

Ferner ist aus der DE 29 27 143 A1 ein chirurgisches Instrument zur Anlage von Nähten bekannt, das insbesondere zum Annähen von Herzschrittmachern dienen soll. Dieses Instrument enthält einen Instrumentenschaft, der ein proximales und distales, in den Körper eines Patienten einzuführendes Ende aufweist. Im Instrumentenschaft verlaufen zwei Kanäle, die Abschnitte eines drahtförmigen Nähguts aufnehmen. Die Kanäle sind am distalen Ende des Instrumentenschafts in der Weise geformt, daß dem Nähgut eine Ringform verliehen wird. Die Elektrode des Herzschrittmachers ist zu Beginn der Operation am distalen Ende des Instrumentenschafts befestigt. Ein zweiteiliges Griffstück dient dem Vorschub des Nähguts und dem Lösen der Elektrode. Das Instrument enthält keine Backen; mit ihm können Wundränder daher allenfalls in günstigen Fällen vernäht werden.

Die US 1,449,087 beschreibt eine zangenartige Nähvorrichtung mit zwei distalen gekrümmten Backen, die jeweils eine Klemmfläche aufweisen. In jeder Backe ist ein gekrümmter Kanal vorhanden, der in der Klemmfläche endet. Mittels eines Kolbens wird eine chirurgische Nähnadel mit Nahtmaterial entlang der gekrümmten Kanäle durch das zwischen den Klemmflächen befindliche Gewebe geschoben.

Die EP-A-0 121 362 offenbart eine Klammervorrichtung, in der ein gerades Stück steifes Nahtmaterial, welches in einem Kanal einer ersten Klemmbacke verschiebbar angeordnet ist, in einem gekrümmten Kanal einer zweiten gegenüberliegenden Klemmbacke etwa kreisringförmig gebogen wird.

Der Erfindung liegt die Aufgabe zugrunde, die Nachteile der bekannten Instrumente zu vermeiden und insbesondere ein chirurgisches Nähinstrument der eingangs genannten Art vorzuschlagen, dessen Backen das Gewebe nicht durchstechen und mit dessen Hilfe mehrere Nähte in einem Arbeitsgang angelegt werden können. Mit dem Instrument sollen sich Wundränder sowohl in der konventionellen Chirurgie als auch in der minimalinvasiven Chirurgie vernähen lassen. Es soll daher durch eine Trokarhülse durchführbar und bedienbar sein.

Die Aufgabe wird erfindungsgemäß durch die im Kennzeichen des ersten Patentanspruchs beschriebenen Merkmale gelöst. In den weiteren Ansprüchen sind vorteilhafte Ausgestaltungen des erfindungsgemäßen chirurgischen Nähinstruments beschrieben.

Mit dem erfindungsgemäßen chirurgischen Nähinstrument können Geweberänder nach einer Läsion oder Inzision fixiert werden. Hierzu werden die beiden zu verbindenden Geweberänder entweder mit einer Faßzange oder mit einer besonderen, im folgenden näher beschriebenen Ausführungsform des erfindungsgemäßen Nähinstruments approximiert und in der Weise miteinander in Kontakt gebracht, daß die beiden auf der gleichen Seite liegenden Flächen des an die Wundränder angrenzenden Gewebes einander berühren. Die Naht wird in der Doppellage des angrenzenden Gewebes angelegt, das annähernd senkrecht in der Art einer Gewebefalte von der Oberfläche des übrigen Gewebes absteht.

Mit den beiden Backen des erfindungsgemäßen Nähinstruments werden die aufeinander liegenden Geweberänder gefaßt. Die beiden Backen können eine Position einnehmen, in der sie annähernd parallel zueinander in einem solchen Abstand voneinander stehen, der etwa der doppelten Dicke des zu vernähenden Gewebes entspricht. Diese Position wird im folgenden als Arbeitsstellung bezeichnet. Je nach Faßtiefe kann in der Arbeitsstellung eine ein- oder doppelreihige Naht gelegt werden.

Die beiden Backen enthalten in ihren einander gegenüberliegenden Seiten ausgehend von dem Kanal zur Zuführung des Nähguts sowohl in der beweglichen Backe als auch in der feststehenden Backe mehrere im Abstand zu einander angeordnete Nuten, deren Grundflächen in der Arbeitsstellung auf einer Schraubenlinie liegen.

Durch die distale Verschiebung des Nähguts in dem Kanal wird das Nähgut aus dem Ende des Kanals in der feststehenden Backe vorgeschoben, durchstößt das doppelt gelegte Gewebe und gelangt schließlich in die erste Nut der beweglichen Backe, wo es umgelenkt wird und beim weiteren Vorschieben des Nähguts (bei ausreichend großer Faßtiefe) das doppelt gelegte Gewebe erneut durchstößt. Danach trifft dessen Spitze die erste Nut der feststehenden Backe, wo das Nähgut auf einer Schraubenlinie weitergeführt wird.

Wenn das Nähgut alle Nuten durchlaufen hat, hat es daher eine schraubenlinien- oder wendelartige Form angenommen. Die Achse der Nähgut-Wendel liegt parallel zu den Wundrändern. Je nach Faßtiefe durchstößt jede Windung der Wendel das Gewebe einoder zweimal, so daß eine einreihige oder zweireihige Naht entsteht. Nach der Fertigstellung der Naht wird das Nähgut abgetrennt und gegen ein Abgleiten vom Gewebe gesichert. Der beschriebene Vorgang wiederholt sich, bis die Geweberänder vollständig miteinander vernäht sind.

Vorzugsweise wird das Nähgut innerhalb des Kanals durch geeignete konstruktive Mittel in einzelnen, kurzen Schritten mit Hilfe des Griffstücks vom proximalen zum distalen Ende bewegt. Die Länge der einzelnen Schritte hängt von der Festigkeit des verwendeten Nähguts ab. Vorzugsweise wird dünner Metalldraht aus Edelstahl, Tantal etc. verwendet. Metalldrähte dieser Art sind ausreichend steif, so daß sie weniger zum Verkanten oder Umbiegen innerhalb des Zuführungskanals neigen; mit ihnen kann außerdem das Gewebe ohne weitere Hilfsvorrichtungen durchstochen werden. Die erfindungsgemäße Vorrichtung kann jedoch auch mit einem anderen, ausreichend steifen Nähgut betrieben werden. Die einzelnen Schritte sollen - entsprechend der mechanischen Belastbarkeit des verwendeten Nähguts - so kurz gewählt werden, daß sich das Nähgut innerhalb des Kanals nicht verkantet und das Gewebe durchsticht, ohne sich umzubiegen; sie sollen jedoch so lang sein, wie unter diesen Bedingungen möglich ist, um den Zeitbedarf für das distale Vorschieben des Nähguts während der Operation in angemessenen Grenzen zu halten. Wenn als Nähgut ein Edelstahldraht mit einem Durchmesser von 0,25 mm verwendet wird, können die einzelnen Schritte etwa 3 mm lang sein.

Vorzugsweise enthält der Kanal eine Sperrvorrichtung, die auf das Nähgut einwirkt und die ein proximales Rückverschieben des Nähguts verhindert, indem das Nähgut nur freigegeben wird, wenn es distal bewegt wird. Besonders bevorzugt ist eine Ausführungsform, bei der die Sperrwirkung z. B. zum Wechseln des Nähguts vom Griffstück aus vom Chirurgen aufgehoben werden kann.

Die bewegliche Backe soll vorzugsweise von der Arbeitsstellung aus sowohl auf die feststehende Backe zu als auch von ihr weg bewegt werden können. In Arbeitsstellung liegen die beiden Backen annähernd parallel zueinander. Ihr Abstand wird konstruktiv vorgegeben und wird im wesentlichen von der doppelten Dicke des zu vernähenden Gewebes bestimmt.

Die Position, in der die bewegliche Backe von der feststehenden Backe weggeklappt ist, wird im folgenden als Ruhestellung bezeichnet. Durch die Bewegung von der Arbeitsstellung in die Ruhestellung und zurück können die zu vernähenden Lagen des Gewebes ergriffen und wieder freigegeben werden.

Die Möglichkeit, die bewegliche Backe ausgehend von der Arbeitsstellung in Richtung auf die feststehende Backe bewegen zu können, ist aus folgenden Gründen vorteilhaft:

Während des distalen Vorschubs des Nähguts beim Nähvorgang wird das Nähgut in eine schraubenlinien- oder wendelartige Form gebracht, denn beim Nähen wird das Nähgut, ausgehend von der in der feststehenden Backe angeordneten Mündung des Kanals, der das Nähgut führt, durch die Nuten in den beiden Backen geführt und geformt. Hierbei wird, wie erwähnt, das zu vernähende Gewebe entweder nur beim Übergang des Nähguts von der feststehenden Backe zur beweglichen Backe oder zusätzlich (bei ausreichender Faßtiefe) beim Übergang von der beweglichen Backe zur feststehenden Backe durchstochen. Die Führung und Formung des Nähguts sowie die Durchstiche durch das Gewebe bewirken eine Reibungskraft, die mit der Zahl der Schraubenlinien oder Wendeln stark ansteigen kann.

Diese Reibungskraft kann durch die Bewegung der beweglichen Backe aus der Arbeitsstellung in Richtung auf die feststehende Backe stark vermindert werden. Der Weg, den die bewegliche Backe hierbei zurücklegt, beträgt vorzugsweise einige Zehntel Millimeter. Diese Bewegung bewirkt, daß der Durchmesser der Wendel des Nähguts geringfügig reduziert wird. Deshalb soll die bewegliche Backe während des Nähvorgangs am Ende eines jeden Vorschubschritts über das Griffstück aus der Arbeitsstellung in Richtung auf die feststehende Backe bewegt werden. In Arbeitsstellung der Backen liegt danach das Nähgut nicht mehr fest auf dem Grund der Nuten auf, so daß die Reibung beim weiteren Vorschub reduziert ist. Die Reibung kann während der Operation durch Zugabe eines geeigneten Gleitmittels zusätzlich vermindert werden. Solche Gleitmittel wie z. B. Siliconöl sind bekannt und werden bei üblichen Nähinstrumenten bereits verwendet.

Durch die Bewegung der beweglichen Backe aus der Arbeitsstellung in Richtung auf die feststehende Backe kann außerdem die fertiggestellte Naht gesichert werden. Nach Beendigung des Nähvorgangs und nach dem Abtrennen des Nähguts wird die bewegliche Backe in die Ruhestellung aufgeklappt, wodurch das Gewebe und die Naht freigegeben werden. Danach wird die Wendel des Nähguts mit den Spitzen der Backen ergriffen und zusammengedrückt, wobei die bewegliche Backe bis in die Arbeitsstellung auf die feststehende Backe zubewegt wird. Hierdurch wird das Aufgehen der Naht verhindert.

Zur Vereinfachung dieses Vorgangs wird eine Ausführungsform besonders bevorzugt, bei der die Spitzen der Backen auf ihren einander gegenüberliegenden Seiten im distalen Bereich vor den Nuten profiliert sind. Eine bevorzugte Profilierung besteht aus eng benachbarten Einsenkungen, die parallel zu den Nuten, d. h. im selben Winkel gegenüber der Achse des äußeren Rohres, angebracht sind und deren Abstände $1/n$ der Abstände zwischen den Nuten entsprechen; n stellt eine ganze Zahl dar. Mit Hilfe dieser Einsenkungen kann die fertiggestellte Wendel des Nähguts besonders sicher erfaßt werden, da die Windungen der Wendel jeweils in einzelne dieser Einsenkungen eingreifen und von diesen abgestützt werden, so daß die Wendel nicht abgleitet und sich nicht unkontrolliert verformt.

Je nach Ausführungsform des erfindungsgemäßen Nähinstruments kann es sinnvoll sein, das äußere Rohr an seinem distalen Ende mit zwei einander gegenüberliegenden Einkerbungen zu versehen. Diese Einkerbungen werden in der Art angebracht, daß das äußere Rohr bei seiner distalen axialen Bewegung relativ zum Instrumentenschaft und damit zu den Backen nicht mit dem zu vernähenden Gewebe in Kontakt kommt und dieses verletzt. Solche Einkerbungen werden vorzugsweise symmetrisch zu einer Ebene angeordnet, die parallel zu derjenigen Seite der feststehenden Backe verläuft, die der beweglichen Backe gegenüber liegt. Die Form der Einkerbungen kann je nach Ausführung des erfindungsgemäßen Nähinstruments in weiten Grenzen variiert werden. Unter Umständen kann das äußere Rohr distal in der Weise abgeschrägt sein, daß die feststehende Backe nicht mehr umfaßt wird.

Das distale Ende des äußeren Rohres und die Ränder der Einkerbungen können zudem in bekannter Weise abgerundet werden, um die Gefahr einer Verletzung des Gewebes weiter zu vermindern.

Der Vorschub des Nähguts beim Nähen wird erleichtert, wenn die Nuten an den Enden, die vom Nähgut zuerst erreicht werden, trichterförmig erweitert sind. Hierdurch wird ein Verkanten des Nähguts an den Nuten verhindert.

Nach Fertigstellung einer Naht kann das Nähgut mit Hilfe eines beweglichen Schlittens mit einer Schneidkante, der in der feststehenden Backe angebracht ist und der von einem Hebel an der proximalen Seite der Vorrichtung aus bewegt wird, abgetrennt werden.

Vorzugsweise wird die bewegliche Backe in folgender Weise aus der Arbeitsstellung von der feststehenden Backe wegbewegt: An der beweglichen Backe ist innerhalb des äußeren Rohres ein im Querschnitt V-förmiges, mit seiner Scheitelkante auf die feststehende Backe weisendes Teil angebracht. Als V-förmiges Teil kann z. B. ein zylindersektorförmiger Körper verwendet werden. Die Scheitelkante des V-förmigen Teils verläuft quer zur Achse des äußeren Rohres und ist in einer quer zur Achse des äußeren Rohres verlaufenden Ausnehmung im Instrumentenschaft gelagert. Am distalen Ende des äußeren Rohres ist auf der der feststehenden Backe gegenüberliegenden Seite ein Stift derart angebracht, daß durch proximales Verschieben des äußeren Rohres relativ zu den Backen die distale Seitenfläche des im Querschnitt V-förmigen Teils durch den Stift proximal verschoben wird, wodurch sich die Scheitelkante in der Ausnehmung dreht und sich die bewegliche Backe von der feststehenden Backe entfernt. Das äußere Rohr ist über das Griffstück gegenüber dem Instrumentenschaft axial verschiebbar.

Die Bewegung der beweglichen Backe in Richtung der feststehenden Backe erfolgt vorzugsweise dadurch, daß das äußere Rohr in distaler Richtung über die bewegliche Backe geschoben wird. Hierdurch wird wieder die Arbeitsstellung, d. h. die parallele Stellung der Backen, erreicht. Die weitere Bewegung der beweglichen Backe aus der Arbeitsstellung auf die feststehende Backe zu wird vorzugsweise durch eine Aufwölbung erreicht, die auf der äußeren Mantelfläche der beweglichen Backe vorgesehen wird und über die das äußere Rohr in einer weiteren distalen Bewegung geschoben wird.

Eine der Backen des erfindungsgemäßen chirurgischen Nähinstruments, z. B. die feststehende, kann auf ihrer der anderen Backe gegenüberliegenden Seite einen spitzen Dorn tragen, der in Arbeitsstellung der Backen in der jeweils anderen Backe in eine entsprechend geformte Vertiefung eingreift.

Mit einem derart modifizierten Instrument kann während des Nähens auf eine Faßzange zum Halten der Geweberänder verzichtet werden. Der Dorn wird in Ruhestellung der Backen an einer geeigneten Stelle unter den ersten Geweberand geführt. Zum Aufladen des Gewebes auf den Dorn werden die Backen in die Arbeitsstellung gebracht, wobei das Gewebe durch den Dorn perforiert wird. Danach wird die bewegliche Backe wieder angehoben (in Ruhestellung gebracht) und in der gleichen Weise der zweite Geweberand erfaßt. Wenn der Dorn im proximalen Bereich der Backen, z. B. hinter der Mündung des Kanals für das Nähgut angeordnet ist, so muß pro Nähvorgang lediglich ein zusätzlicher Durchstich toleriert werden. Wenn der Dorn im distalen Bereich an der Spitze der Backen angeordnet ist, erleichtern bessere Sichtverhältnisse das Greifen der Wundränder. In diesem Fall muß bei der abschließenden Deformation des wendelförmigen Nähguts ein weiterer Durchstich toleriert werden.

Die Erfindung wird im folgenden anhand von Figuren, in denen eine besondere Ausführungsform des erfindungsgemäßen chirurgischen Nähinstruments dargestellt ist, näher erläutert.

Es zeigen

Fig. 1     eine Gesamtansicht einer Ausführungsform des erfindungsgemäßen Nähinstruments;

Fig. 2     den Kanal für das Nähgut und die Vorrichtung zu dessen distalem Vorschub;

Fig. 3a    das distale Ende des Instruments mit den Backen und der Sperrvorrichtung;

Fig. 3b    die feststehende Backe mit dem beweglichen Schlitten der Schneidvorrichtung in Aufsicht;

Fig. 4     den Griffkopf im Teilschnitt;

Fig. 5     eine weitere Ansicht des Griffkopfs;

Fig. 6     den Instrumentenschaft;

Fig. 7     die Schneidvorrichtung.

Die gezeigte Ausführungsform des erfindungsgemäßen Nähinstruments ist für einen Edelstahldraht von 0,25 mm Durchmesser als Nähgut konzipiert; sie besteht aus drei Baugruppen:

- dem Arbeitskopf mit den Backen 23, 24 und der Schervorrichtung 45 für das Nähgut 17,
- dem Griffstück 1, 2 bestehend aus Griffkopf 18 mit starr angeschlossenem Handtellerteil 2 und dem relativ hierzu beweglichen Fingerteil 1 und
- dem Instrumentenschaft 20, der die Verbindung zwischen den Backen 23, 24 und dem Handtellerteil 2 darstellt, sowie dem äußeren Rohr 21 zur Betätigung der beweglichen Backe 24 vom Fingerteil 1 aus.

Ferner kann zwischen folgenden Funktionen unterschieden werden, die mit Teilen ihrer mechanischen Realisierung z. T. in allen drei Baugruppen zu finden sind:

- Betätigung der beweglichen Backe 24,
- Ausführungsform mit zusätzlichem Dorn: Aufladen und Zusammenführen der Geweberänder,
- Formen des Nähguts 17 zu einer Wendel 26,
- Verminderung des Durchmessers der Wendel des Nähguts 17 z. B. nach jedem Vorschubschritt zur Verminderung der Reibung zwischen Nähgut 17 und den formgebenden und führenden Nuten 38 in den Backen 23, 24,
- wiederholtes Klemmen und anschließender schrittweiser Vorschub des Nähguts 17 durch die Vorschubvorrichtung 14, 28,
- selbsthemmende Arretierung des Drahts 17 gegen Rückzug in der Sperrvorrichtung 57, 58 und bei der Repositionierung der Klemm- und Vorschubeinrichtung 14, 28 in die Ausgangsstellung,
- Trennen des z. B. von der Rolle ablaufenden Nähguts am Nahtende durch den beweglichen Schlitten 45,
- Umbiegen des Nähguts 17 am Nahtanfang und -ende zum Gewebe hin, um nach dem Aufklappen der beweglichen Backe 24 den Drahtrückzug ins Gewebe und das Aufgehen der gesetzten Naht vor der abschließenden Sicherung zu erschweren,
- Formen des Nähgut-Anfangs beim Abschneiden, so daß die Durchstichkraft minimiert wird und beim Gewebedurchstich auf die Spitze des Nähguts eine Führungskraft zur Wendelachse hin erzeugt wird,
- Verformung des zunächst kreisförmigen Nähgutwendel-Querschnitts in ein flaches Oval als Sicherung gegen Aufziehen der Naht,
- Sicherung der Schermechanik 45 gegen unbeabsichtigte Betätigung und
- Modifizierung der Funktionssequenzen durch verschiedene Stellschrauben 15, 16 bei vorgegebener Betätigung des Griffstücks 1, 2.

Das distale Ende des äußeren Rohres 21 mit dem umfaßten Instrumentenschaft 20 wird durch eine 10 mm - Trokarhülse in den Körper des Patienten eingeführt. Dazu wird während des Durchschiebens die in Ruhestellung hochgeklappte bewegliche Backe 24 durch einen kurzen Weg am Fingerteil 1 des Griffstücks 1, 2 und der damit gekoppelten translatorischen Bewegung des äußeren Rohres 21 in die Arbeitsstellung parallel zur feststehenden Backe 23 gebracht.

Alle übrigen Instrumentenfunktionen werden dabei wegen eines einstellbaren Leerweges in der Koppelmechanik nicht aktiviert.

Die Konstruktion des Instruments ist so gewählt, daß zur Erleichterung einer exakten Manipulation im Operationsfeld der Abstand zwischen dem handgeführten festen Griffteil 2 und den beiden Backen 23, 24 konstant bleibt. Beim Betätigen der beweglichen Backe 24 wird lediglich das äußere Rohr axial verschoben, so daß zur Beibehaltung einer gewünschten räumlichen Position der Backen 23, 24 keine kompensierende Handbewegung des Chirurgen erforderlich ist.

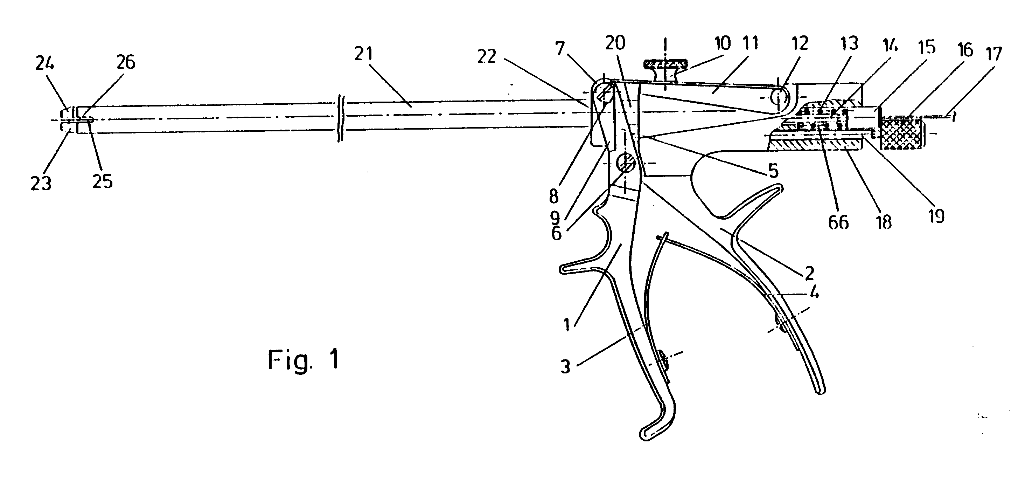

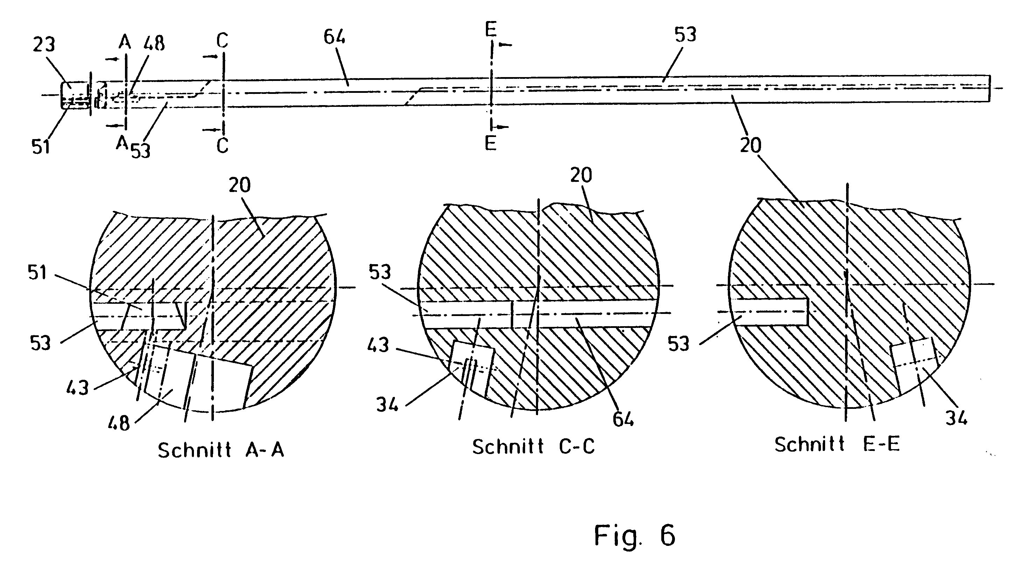

Das distale Ende des Instruments besteht aus den beiden Backen 23, 24. Die als feststehend bezeichnete Backe 23 wird durch entsprechende Formung des distalen Endes des Instrumentenschafts hergestellt (Fig. 1 und 6).

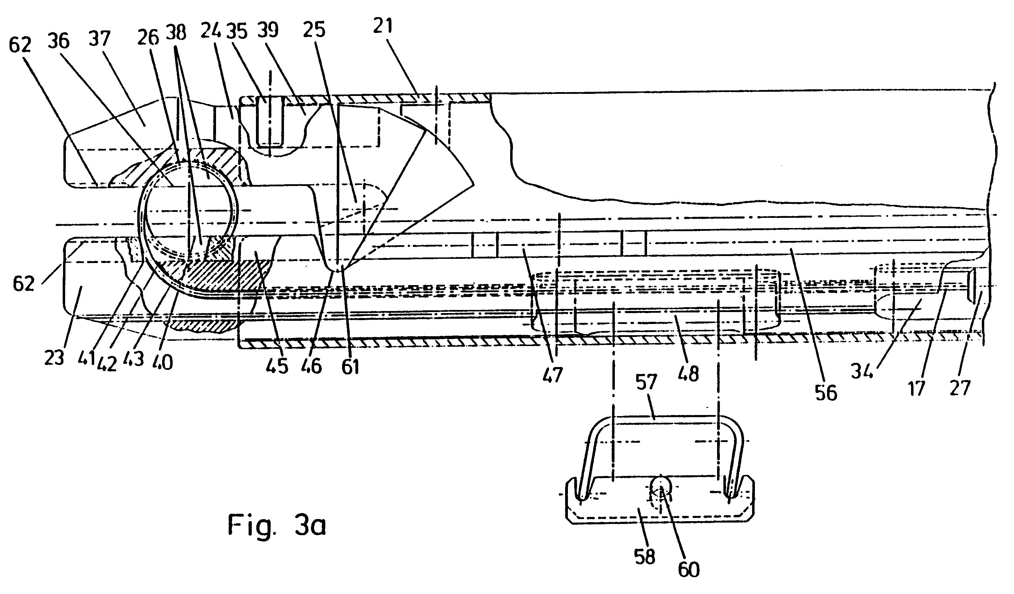

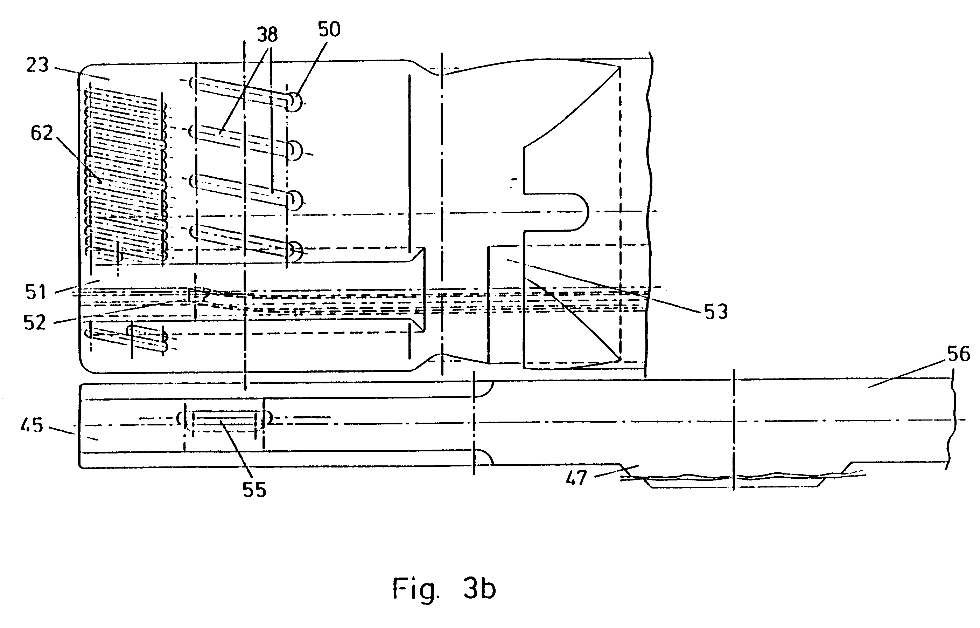

Die Konstruktion der Backe 24 und die Führung des Nähguts 17 sind aus den Fig. 3a und 3b ersichtlich.

Der Drehpunkt der zweiten, beweglichen Backe 24 liegt in einer Ausnehmung 46 des Instrumentenschafts 20 hinter der feststehenden Backe 23.

Das Aufklappen der beweglichen Backe 24 geschieht durch einen Stift 35 in dem auf dem Instrumentenschaft 20 verschiebbaren äußerem Rohr 21, während das Zuklappen durch das Übergleiten der oberen distale Vorderkante des äußeren Rohrs 21 auf die äußere Backenfläche erfolgt. Die Positionssicherung der beweglichen Backe 24 relativ zur festen Backe 23 ist nur durch die Geometrie ihres Einschlusses gewährleistet, und zwar durch die Lage in der Ausnehmung 46 des Instrumentenschafts 20 und durch die Einbettung in das äußere Rohr 21. Auf Stifte und Verbindungs- oder Lagerbolzen wurde verzichtet, da sie aufgrund ihrer notwendigerweise kleinen Abmessungen schwierig anzubringen und häufig überlastet sind.

Wie bereits erwähnt, werden die beiden aufeinanderliegenden Geweberänder so mit den Backen 23, 24 gefaßt, daß

- entweder nur eine Durchstichreihe dicht hinter ihnen entsteht und das fortlaufende Nähgut 17 außen an ihnen vorbeiläuft oder daß
- bei weiterem Übergreifen der Backen 23, 24 zwei parallele Durchstichreihen erzeugt werden und der Nähdraht nur noch an der Ober- und Unterseite der Naht sichtbar bleibt.

Die schraubenförmige Nahtwendel wird schrittweise durch mehrmaliges Betätigen des Griffstücks 1, 2 (Fig. 1) erzeugt. Die Wendel mit Kreisquerschnitt hat bei einem Nähgut aus 0,25 mm starkem Draht einen Außendurchmesser von 4 mm.

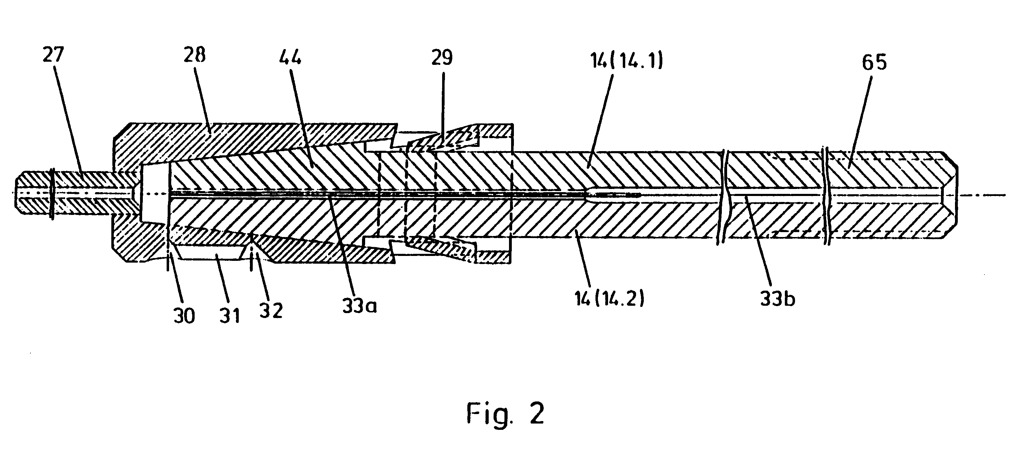

Über einen weiter unten beschriebenen Drahtklemm- und Vorschubmechanismus 14, 28 (vgl. Fig. 2) wird, nachdem die bewegliche Backe 24 in Arbeitsposition gebracht wurde, der Draht durch weiteres Zusammendrücken des Griffstücks 1, 2 aus dem Führungskanal 43 distal zur Mündung in der festen Backe 23 vorgeschoben. Der Vorschub erfolgt schrittweise mit jeweils 3 mm. Das Nähgut 17 tritt zunächst durch einen der Wendelsteigung angepaßten Schlitz 55 des beweglichen Schlittens 45 in der festen Backe 23 hindurch.

In dem Bereich des Führungskanals 43, der in der festen Backe 23 und im beweglichen Schlitten 45 liegt, erhält der Draht 17 zunächst eine Bogenformung in einer Ebene parallel zur Instrumentenlängsachse. Der Radius dieses Bogens ist aus Platzgründen (Unterbringung des beweglichen Schlittens 45 in der festen Backe 23) etwas größer als der Radius der fertigen Wendel, so daß erst nach dem ersten Gewebedurchstich die endgültige Radiusformung des Drahtes 17 in der beweglichen Backe 24 stattfinden kann.

Am Ende jedes Drahtvorschub- und Formungstaktes läuft das distale Ende des äußeren Rohres 21 auf eine exzentrisch auf dem Mantel der beweglichen Backe liegenden, aufgewölbten Querrippe 37. Dadurch werden die Backen 23, 24 jeweils 0,3 mm enger zusammengeführt, was eine entsprechende Durchmesserreduzierung der Nahtwendel zur Folge hat.

Diese Maßnahme ist erforderlich, um ein Festlaufen der gebildeten Wendel in den Führungsnuten 38 der Backen 23, 24 infolge der Gesetzmäßigkeit

$$S_2 = S_1 \cdot \exp(\mu\alpha) \qquad (1)$$

mit

$S_2$ = Vorschubkraft für den Draht [N]
$S_1$ = Reibungs- und Widerstandskraft an der Drahtspitze [N]
$\mu$ = Reibungskoeffizient zwischen Draht und Führungsnut[-]
$\alpha$ = Berührungswinkel von Draht und Nut [rad]

der Seilreibung zu verhindern.
(Die Reibung im Gewebe ist in Gl. (1) vernachlässigt.)

Um ein sicheres Einspuren der Drahtspitze nach jedem Gewebedurchstich zu fördern, sind die entsprechenden Einläufe 50 der Führungsnuten 38 konisch erweitert (siehe Fig. 3b).

Zur vollständigen oder teilweisen Ausführung der Wendelnaht 26 sind z Arbeitshübe bei parallel liegenden Backen 23, 24 erforderlich. Die Zahl z ist eine Funktion der gewünschten Nahtlänge bzw. der gewünschten Stich- oder Windungszahl und des gewählten Hubs des Drahtvorschubes.

Am Ende jeder Teilnaht wird das Griffstück 1,2 bis zum Anschlag des Arbeitsbereiches "Wendelverengung" angezogen. Damit wird der daumenerreichbare Hebel 11 am Griffkopf 18 (vgl. Fig. 1) zur Betätigung freigegeben. Durch Niederdrücken dieses Hebels 11 wird der bewegliche Schlitten 45 in der Schwalbenschwanznut 51 der festen Backe 23 nach proximal verschoben, der Draht 17 im Scherauge 55 an der Scherkante 41 abgeschert und das Wendelende durch die verrundete Oberkante des Schlittens 45 in die Ebene der Backeninnenflächen 36, 40 umgebogen.

Diese Maßnahme dient ebenso wie eine entsprechende Drahtformung am "Kopfende" einer vollständigen Naht der Sicherung gegen "Aufziehen" der Wendel nach Öffnen der beweglichen Backe 24 z.B. zum Umsetzen der Backen 23, 24. Dieses Umsetzen ist vorgesehen, um - falls erforderlich - den kreisrunden Wendelquerschnitt im Nachformungsbereich des vorderen Backenbereichs flachoval zu verformen. Das Ergebnis ist eine Erhöhung der Sicherheit gegen "Aufziehen" der Naht.

Beim Trennen des Drahtes 17 am Wendelende wird durch Formgebung der Schneidkante 52 in der festen Backe - zwei Schneiden im Winkel von 60° zueinander - der Drahtanfang unter den gegebenen Möglichkeiten so geformt, daß

- der Durchstichwiderstand im Gewebe möglichst klein ist und

- beim Gewebedurchstich eine Kraftkomponente in radialer Richtung zur Wendelachse hin auftritt, die ein zentrifugales Ausbrechen des Drahtes 17 infolge des aufbiegenden Momentes verhindert.

Wie bereits beschrieben, wird der Instrumentenschaft 20 vom äußeren Rohr 21 umgeben, das durch Betätigen des Griffstücks 1,2 axial auf dem Instrumentenschaft verschiebbar ist.

Im Instrumentenschaft 20 (siehe Fig. 6), der am distalen Ende zur festen Backe und zur Lagerung der beweglichen Backe ausgebildet ist, befinden sich die Längsnuten 51, 53 für die Aufnahme und Führung des beweglichen Schlittens 45 und und seiner Verbindungsstange 56 zum Antrieb des Schlittens der Schervorrichtung sowie die Längsnut 34 zur Führung des Stützröhrchens 27 für den Nähdraht 17. Ferner ist in diesem Instrumententeil im Verlauf des quadratischen Führungskanals 43 des Drahtes - in Vorschubrichtung dicht vor der festen Backe 23 - eine Tasche 48 zur Aufnahme einer federbelasteten Bremsbacke 58 vorhanden.

Da die Drahtklemm- und Vorschubeinrichtung 14, 28 (vgl. hierzu Fig. 2) aus Platzgründen im Griffkopf 18 des Instrumentes untergebracht ist, muß der Nähdraht (0,25 mm $\varnothing$) gegen Ausknicken gesichert durch den Instrumentenschaft 20 bis zum Ende des Führungskanals 43 in der festen Backe 23 geschoben werden. Zu diesem Zweck wird er während des Vorschubes in einem Drahtstützrohr 27 (Fig. 2 und 3a) mit 1,5 mm Außendurchmesser und 0,4 mm Innendurchmesser knickfrei gehalten. Das Drahtstützrohr 27 ist mit der Klemmhülse 28 der Vorschubmechanik verschweißt und wird entsprechend dem gewählten Vorschub translatorisch in der Nut 34 des Instrumentenschaftes 20 hin und her bewegt.

Beim Zurückfahren des Drahtstützrohres 27 verhindert die federbelastete Bremsbacke 58 (Fig. 3a) durch Eintritt von Selbsthemmung ein Zurückziehen des Nähdrahtes 17 infolge der Reibung an der Rohrinnenwand und im gelösten Klemmbolzen 14 (Fig. 1, 4 bzw. 14.1, 14.2 in Fig. 2).

Der Chirurg kann - z. B. zum Wechseln des Nähguts 17 - durch einen Steckstift in der Bohrung 60 der Bremsbacke 58 und einer entsprechenden Bohrung im äußeren Rohr 21 eine Kopplung zwischen diesen Teilen herstellen und durch Betätigen des Griffstücks 1, 2 die Sperrwirkung aufheben.

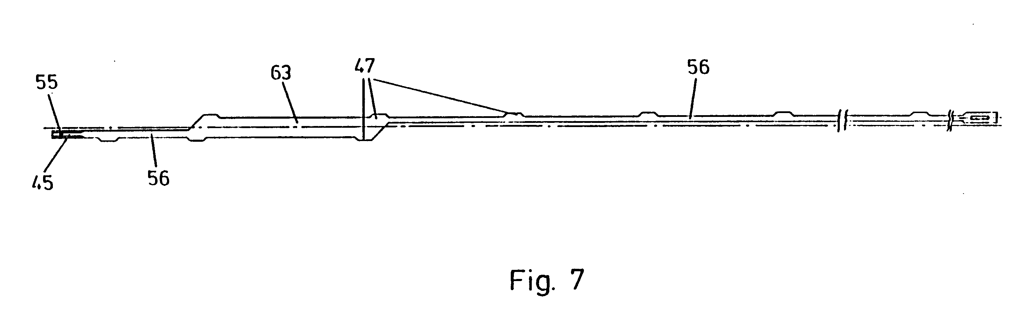

Der bewegliche Schlitten 45 und die Verbindungsstange 56 (Fig. 3b, Fig 7) des Schlittenantriebes sind einteilig aus partiell härtbarem nichtrostendem Stahlblech gefertigt. Bedingt durch die geometrischen Gegebenheiten im Griffkopf 18 wurde durch einen Versatz in der Verbindungsstange 56 ein Seitenwechsel vom Schlittenoberteil 45 in der festen Backe 23 zum Schlittenantrieb in einer Tasche 59 des Griffkopfes 18 realisiert. Das Etagenstück 63 in der Verbindungsstange 56 (Fig. 7) ist relativ lang ausgeführt, um die bei Belastung auftretenden Richtmomente durch kleine Stützkräfte zwischen den Führungsnocken 47 dieses Teiles und der Innenwand des äußeren Rohres 21 kompensieren zu können. Der Querdurchbruch 64 für das Etagenstück 63 ist mit Rücksicht auf die Montage um die Länge der Schwalbenschwanznut 51 in der festen Backe 23 nach proximal erweitert.

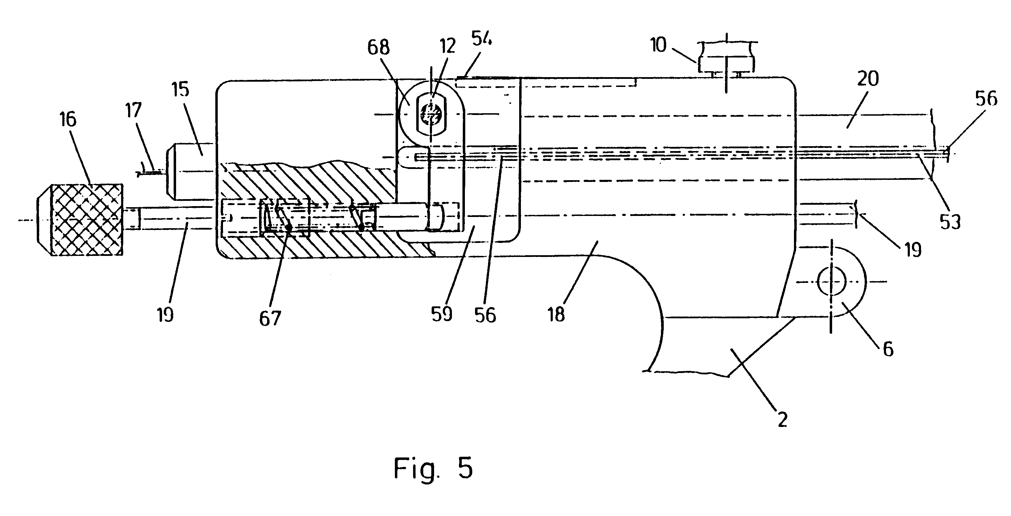

Das Griffstück 1, 2 (vgl. Fig. 1, 4 und 5) des Nähinstruments besteht aus

- dem Griffkopf 18,
- dem relativ zu ihm beweglichen Fingergriffteil 1 und
- dem über den Griffkopf 18 starr mit dem Instrumentenschaft 20 verbundenen Handteller-Griffteil 2.

Die beiden Griffteile 1, 2 werden durch zwei zwischen ihnen angeordnete Flachfedern 3, 4 in üblicher Weise in Ruhestellung maximal gespreizt.

Der Griffkopf 18 dient

- zur beweglichen Lagerung des Fingergriffteiles 1,
- Unterbringungen der Klemm- und Vorschubeinrichtung 14, 28 für den Nähdraht 17,
- zur Installation des Schlittenantriebes und
- zur Verbindung des Handteller-Griffteiles 2 mit dem Instrumentenschaft 20.

Der Handbereich des Fingergriffteiles 1 ist in üblicher Formgebung griffsympatisch ausgeführt. Die sich nach oben anschließende Gabel 5 ist zur Bildung der Lagerstelle mit dem Lagerzapfen 6 am Griffkopf 18 gelenkig verschraubt. Ferner sind die oberen Enden der Gabel 5 gelenkig mit dem am proximalen Ende des äußeren Rohres 21 angeschweißten Rechteckflansch 22 durch Schrauben mit abgesetztem Schaft 7 verbunden. Die Schäfte der Schrauben 7 gleiten bei der translatorischen Verschiebung des äußeren Rohres 21 in Langlöchern 8 am Gabelende. Die dem daumenbetätigten Hebel 11 zugewandte Gabelwange ist partiell verdickt. Diese Blockadekulisse 9 dient im normalen Arbeitsbereich des Griffstücks 1,2 als Sicherung gegen unbeabsichtigtes Betätigen des beweglichen Schlittens 45.

Die Bewegung des Rechteckflansches 22 (und des äußeren Rohres 21) wird über eine im Griffkopf 18 geführte Koppelstange 19 teilweise auf die Klemm- und Vorschubmechanik 14, 28 für den Nähdraht 17 übertragen. Durch einen gezielt einstellbaren Leerweg (Hutmutter 16 und Spannmutter 15) findet eine Kraftübertragung auf den Klemmbolzen 14.1, 14.2 erst statt, wenn die bewegliche Backe 24 durch kurzwegige initiale Verschiebung des äußeren Rohres 21 bereits in der parallelen Arbeitsposition ist.

Die Klemm- und Vorschubmechanik 14, 28 (vgl. Fig. 2) besteht aus zwei Hauptkomponenten:

- dem Außenteil 28 (Klemmhülse) mit konischer Bohrung und zwei einander gegenüberliegenden nach innen gebogenen Mitnehmerzungen 29 sowie dem stirnseitig koaxial eingeschweißten Drahtstützrohr 27 zur Stützung des Nähdrahtes 17 und

- dem Innenteil (Klemmbolzen 14) mit dem geschlitztem Konus 44 zur reibschlüssigen Klemmung des Drahtes 17 und dem längsgeteilten Gewindebereich 65 zur Einleitung der Vorschubkraft.

Beide Teile liegen in einer Stufenbohrung 66 des Griffkopfes 18 in achsfluchtender Verlängerung des Drahtführungskanals 43, 14 im Innenteil des Instrumentenschaftes 21.

Der Außenmantel der Klemmhülse 28 trägt im Abstand von 3 mm in Längsrichtung zwei konische Bohrungen 30, 32, die durch eine weniger tiefe Dreikantnut 31 miteinander verbunden sind. Ein flachfederbelasteter Stift 49 im Griffkopf 18 fixiert die Klemmhülse 28 kraft- und formschlüssig entsprechend den Positionen der konischen Bohrungen 30, 32 im Mantel der Hülse 28. Beim Verschieben der Hülse 28 gleitet der federbelastete Stift 49 durch die Verbindungsnut 31 zwischen den Bohrungen 30, 32 und verhindert so ein Verdrehen der Hülse 28 und damit eine unerwünschte Torsion des Drahtes 17. Durch den Abstand der Bohrungen 30, 32 im Mantel ist der Drahtvorschubweg festgelegt. Weitere Kulissen mit anderen Abständen können jedoch in gleicher Weise nebeneinander auf dem Hülsenmantel angebracht werden. Der Wunsch nach einem längeren oder kürzeren Vorschubweg kann dann durch einfaches Drehen der Hülse auf andere Kulissenbahnen erfüllt werden.

Der Klemmbolzen 14 ist aus Fertigungsgründen zunächst axial geteilt. Beide Hälften 14.1, 14.2 werden nach dem Fräsen der prismatischen Innennuten 33a, 33b in der Achse des Klemmbolzens 14 miteinander verschweißt. Konus 44 - und Gewindeende 65 können schwach aufgebogen werden, um eine sichere Drahtfreigabe im Klemmbereich 33a bzw. eine reibschlüssige Mutterblockade auf dem Gewinde 65 zu erreichen.

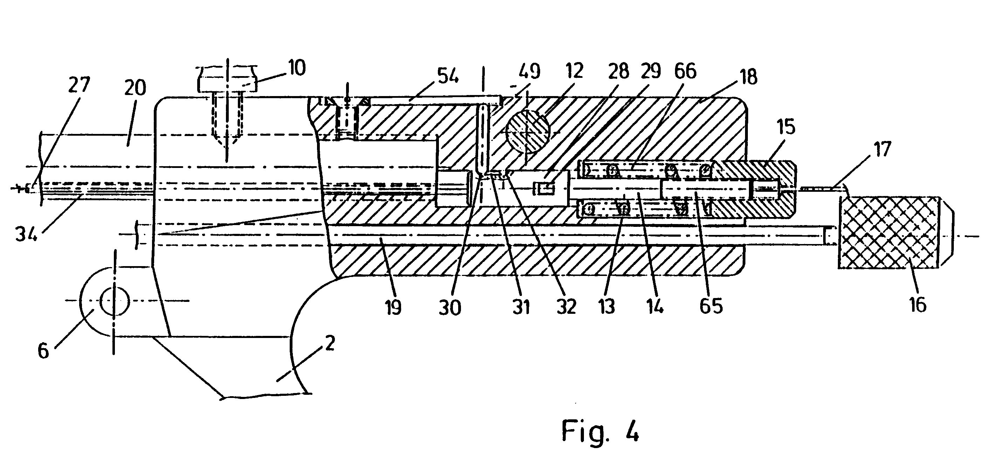

Zwischen der Stellmutter 15 (Fig. 4; zur Feineinstellung der Federkraft) auf dem Gewinde 65 des Klemmbolzens 14 und dem Bankett der abgesetzten Aufnahmebohrung 66 für die Vorschubmechanik 14, 28 (vgl. Fig. 2) ist eine starke Schraubendruckfeder 13 angeordnet. Sie drückt in Ruhestellung des Instruments den Klemmbolzen 14 nach außen, löst damit den Klemmbolzenkonus 44 vom Draht 17 und zieht die Klemmhülse 28 soweit zurück, daß der federbelastete Arretierstift 49 in der distalen konischen Bohrung 30 im Hülsenmantel 28 einrastet.

Beim Betätigen des Griffstücks 1, 2 aus seiner Ruhelage heraus wird - wie beschrieben - zunächst die bewegliche Backe in die parallele Arbeitsstellung gebracht. Danach drückt die Leerweg-Einstellmutter 16 auf der Koppelstange 19 im Griffkopf 18 gegen die Spannmutter 15 des Klemmbolzens 14. Der Konus 44 des Klemmbolzens 14 wandert in die Klemmhülse 28, wird zusammengedrückt und klemmt in seinem axialen Prismenkanal 33a den durch die Zentralbohrung der Spannmutter 15 zugeführten Draht 17 fest. Bei weiterer Erhöhung der Schubkraft wird der Arretierstift 49 im Griffkopf 18 gegen den Druck der Flachfeder 54 aus der distalen Bohrung 30 in der Klemmhülse 28 herausgehoben.

Die Klemmhülse 28 einschließlich Klemmbolzen 14 und Nähdraht 17 wird - durch den Stift in der Dreikantnut 31 zwischen den Bohrungen 30, 32 gegen Verdrehen gesichert - nach distal verschoben, bis am Ende des Vorschubweges der Stift 49 in der proximalen Senkung 32 der Kulisse 30, 31, 32 einrastet.

In dieser Position ist die Vorderkante des äußeren Rohres 21 auf die exzentrische Querrippe 37 der beweglichen Backe 24 geschoben und damit eine Radiusverkleinerung der Drahtwendel erreicht.

Wird die Handkraft auf die Griffteile vermindert, bewegen sie sich durch die Wirkung der beiden zwischen ihnen angeordneten Blattfedern 3, 4 auseinander. Das äußere Rohr wird auf dem Instrumentenschaft 20 nach proximal zurückgezogen. Die gespannte Klemmbolzenfeder 13 zieht den Klemmbolzen 14 unter Freigabe des Nähdrahtes 17 aus dem Innenkonus der noch durch den Arretierstift 49 blockierten Klemmhülse 28 und zieht ihn bis zu den beiden Anschlagzungen 29 in der Klemmhülse 28 zurück. Jetzt wirkt die Kraft der Klemmbolzenfeder 13 auch auf die Klemmhülse 28. Dadurch gleitet der Arretierstift 49 aus der proximalen Bohrung 32 in die Dreikantnut 31 der zurückbewegten Klemmhülse 28 und schließlich in die distale Bohrung 30.

Ein Rückzug des Nähdrahtes 17 bei diesem Vorgang durch die Reibung zwischen Draht und Drahtstützröhrchen 27 wird durch die auf den gezogenen Draht 17 selbsthemmend wirkende Bremsbacke 58 in Backennähe verhindert.

Aus der nun erreichten Position - bei parallelen Backen 23, 24 - kann durch erneute Betätigung des Griffstücks 1, 2 ein weiterer Zyklus mit Vorschub und Rückzug des Vorschubeinrichtung 14, 28 eingeleitet werden.

Nach ca. 16 bis 17 Zyklen sind etwa 4 Nahtwindungen komplett. Bei vollständig zusammengedrücktem Handgriff wird mit dem Daumen derselben Hand der Hebel 11 niedergedrückt und damit die Wendel vom restlichen Nähdraht getrennt.

Nach Rückstellung des beweglichen Schlittens 45 über den durch die Feder 47 belasteten Hebel 68 kann das Griffstück 1, 2 bis zum Endanschlag geöffnet werden. Dadurch bewegt sich die bewegliche Backe in Ruhestellung.

Nach entsprechendem Umsetzen des Instrumentes können die Backen 23, 24 zur flachovalen Umformung der Wendel in ihrem vorderen Bereich erneut geschlossen werden. Diese Schließbewegung findet nur im Rahmen des Freilaufweges, also ohne Aktivierung der Klemm- und Vorschubmechanik statt.

Zur Erleichterung der Umformung sind die Innenflächen 36, 40 im distalen Teil der Backen 23, 24 durch Einsenkungen 62 profiliert.

Bezugszeichenliste

| | |
|---|---|
| 1 | Fingergriffteil, Griffstück |
| 2 | Handtellergriffteil, Griffstück |
| 3 | Flachfeder 1 |
| 4 | Flachfeder 2 |
| 5 | Gabelkopf des Fingergriffteiles |
| 6 | Lagerzapfen |
| 7 | Koppelbolzen zum Rechteckflansch, Schrauben mit abgesetztem Schaft |
| 8 | Langloch in den Gabelkopfschenkeln |
| 9 | Blockadekulisse für Scherenhebel |
| 10 | Klemmschraube für Instrumentenschaft |
| 11 | daumenbetätigter Scherenhebel |
| 12 | Hebelwelle |
| 13 | Rückzugfeder der Klemm- und Vorschubeinrichtung für den Nähdraht |
| 14 | Klemmbolzen, gefügt |
| 14.1 | Klemmbolzen, 1. Hälfte |
| 14.2 | Klemmbolzen, 2. Hälfte |
| 15 | Hutmutter mit Zentraldurchgangsbohrung |
| 16 | Hutmutter, gerändelt |
| 17 | Nähdraht, Nähgut |
| 18 | Griffkopf |
| 19 | Koppelstange |
| 20 | Instrumentenschaft |
| 21 | äußeres Rohr |
| 22 | Rechteckflansch |
| 23 | feste Backe |
| 24 | bewegliche Backe |
| 25 | Einkerbungen, Gabelöffnung im äußeren Rohr |
| 26 | Nahtwendel |
| 27 | Drahtstützröhrchen |
| 28 | Klemmhülse |
| 29 | Mitnehmerzunge |
| 30 | distale Indexbohrung |
| 31 | Führungskanal und Verdrehsicherung, Dreikantnut |
| 32 | proximale Indexbohrung |
| 33a | Klemmkanal, prismatisch |
| 33b | Führungskanal, prismatisch |
| 34 | Führungsnut für Stützröhrchen 27 |
| 35 | Stift für Maulöffnung |
| 36 | Fläche der beweglichen Backe |
| 37 | Aufwölbung, Exzenterteil der beweglichen Backe |
| 38 | Nut zur Wendelformung |
| 39 | Nut für Schließhülsenstift |
| 40 | Fläche der festen Backe |
| 41 | Schneide |
| 42 | Scherenschneide in der festen Backe |
| 43 | Führungskanal |
| 44 | Klemmbolzen-Konus |
| 45 | Scherenblatt, beweglicher Schlitten |
| 46 | Lager der beweglichen Backe |
| 47 | Führungsnocken am Scherenschaft 56 |
| 48 | Gehäuse für Drahtrücklaufbremse im Instrumentenschaft |
| 49 | Arretierstift |
| 50 | Einlauftrichter |
| 51 | Schwalbenschwanznut für Scherenblatt |
| 52 | Nähdrahtaustritt in der festen Backe mit V-förmiger Schneide |
| 53 | Führungsnut für Scherenschaft |
| 54 | Flachfeder für Arretierstift |
| 55 | Drahtführungsschlitz mit Schneide im Scherenblatt |
| 56 | Verbindungsstange, Scherenschaft |
| 57 | Rückstell- und Andruckfeder der Bremsbacke zur Drahtrücklaufblockade |
| 58 | Bremsbacke |
| 59 | Tasche für Scherenmechanik |
| 60 | Backenbohrung zur Aufhebung der Drahtblockade (bei gewolltem Drahtrückzug) |
| 61 | V-förmige Lagerkante der beweglichen Backe |
| 62 | Einsenkung, Profilierung für Wendelnachformung |
| 63 | Etagenstück im Scherenschaft 56 |
| 64 | Querdurchbruch im Instrumentenschaft 20 |
| 65 | längsgeteilter Gewindebereich des Klemmbolzens |
| 66 | Stufenbohrung im Griffkopf |
| 67 | Rückstellfeder für Scherenblatt |
| 68 | Zwischenhebel im Scherenantrieb |

**Patentansprüche**

1. Chirurgisches Nähinstrument

a) mit einem Instrumentenschaft (20), der ein distales, in den Körper eines Patienten einzuführendes Ende und ein proximales Ende aufweist;

b) mit zwei einander gegenüberliegenden Backen (23, 24) am distalen Ende des Instrumentenschafts (20), wobei

c) die erste Backe (23) fest mit dem Instrumentenschaft (20) verbunden ist und

d) die zweite Backe (24) gegenüber der ersten Backe (23) beweglich am Instrumentenschaft (20) angeordnet ist,

e) mit einem Kanal (43) im Instrumentenschaft (20) zur Zuführung eines Nähguts (17) vom proximalen Ende zum distalen Ende, der auf derjenigen Seite (40) der ersten, feststehenden Backe (23), die der zweiten, beweglichen Backe (24) gegenüberliegt, endet,

f) mit einem gegenüber dem Instrumentenschaft (20) axial verschiebbaren äußeren Rohr (21), das zwischen dem distalen und dem proximalen Ende des Instrumentenschafts (20) angeordnet ist, diesen umschließt, die Backen (23, 24) jedoch zumindest teilweise freiläßt,

g) mit einem mehrteiligen Griffstück (1, 2) am proximalen Ende des Instrumentenschafts (20) zur Betätigung der bewegliche Backe, von dem ein Teil (2) mit dem proximalen Ende des Instrumentenschafts (20) und ein weiteres Teil (1) mit dem äußeren Rohr (21) verbunden ist,

dadurch gekennzeichnet, daß

h) die beiden Backen (23, 24) eine Stellung zueinander einnehmen können, in der sie über ihre gesamte Länge mit Abstand annähernd parallel zueinander angeordnet sind und
i) in ihre einander gegenüberliegenden Seiten (36, 40) ausgehend von dem Kanal (43) zur Zuführung des Nähguts (17) sowohl in der beweglichen Backe (24) als auch in der feststehenden Backe (23) mehrere im Abstand zu einander angeordnete Nuten (38) eingebracht sind, deren Grundflächen in dieser Stellung auf einer Schraubenlinie liegen.

2. Chirurgisches Nähinstrument nach Anspruch 1, gekennzeichnet durch Mittel (14, 28), mit deren Hilfe das Nähgut (17) durch den Kanal (43) über das Griffstück (1, 2) schrittweise distal verschiebbar ist in der Weise, daß es vom Ende des Kanals zuerst in die erste der Nuten (38) der beweglichen Backe (24), danach in die erste der Nuten (38) der feststehenden Backe (23) und schließlich abwechselnd in die weiteren Nuten der beweglichen (24) und der feststehenden (23) Backe gelangt.

3. Chirurgisches Nähinstrument nach Anspruch 2, gekennzeichnet durch eine Sperrvorrichtung (57, 58), die auf das Nähgut (17) in dem Kanal (43) einwirkt, und die das Nähgut (17) nur bei distaler Verschiebung freigibt.

4. Chirurgisches Nähinstrument nach Anspruch 1 oder 2, dadurch gekennzeichnet, daß die bewegliche Backe (24) über das Griffstück (1, 2) ausgehend von der parallelen Stellung sowohl auf die feststehende Backe (23) zu als auch von ihr weg bewegbar ist.

5. Chirurgisches Nähinstrument nach Anspruch 1 oder 4, dadurch gekennzeichnet, daß das äußere Rohr (21) an seinem distalen Ende zwei einander gegenüberliegende Einkerbungen (25) aufweist, die symmetrisch zu einer Ebene angeordnet sind, die parallel zu der Seite (40) der feststehenden Backe (23), die der beweglichen Backe (24) gegenüber liegt, verläuft.

6. Chirurgisches Nähinstrument nach Anspruch 1, dadurch gekennzeichnet, daß die beiden Backen (23, 24) auf ihren einander gegenüberliegenden Seiten (36, 40) im distalen Bereich vor den Nuten (38) eng benachbarte Einsenkungen (62) aufweisen, die parallel zu den Nuten (38) verlaufen und deren Abstand voneinander 1/n des Abstandes der Nuten (38) beträgt, wobei n eine ganze Zahl darstellt.

7. Chirurgisches Nähinstrument nach Anspruch 1 oder 2, dadurch gekennzeichnet, daß die Nuten (38) an den Enden, die durch das Nähgut (17) bei dessen distaler Verschiebung zuerst erreicht werden, in der Art eines Trichters (50) erweitert sind.

8. Chirurgisches Nähinstrument nach einem der Ansprüche 1 bis 7, dadurch gekennzeichnet, daß die feststehende Backe (23) einen bewegbaren Schlitten (45) mit einer Schneidkante (41) enthält, mit der das Nähgut (17) abgetrennt werden kann.

9. Chirurgisches Nähinstrument nach Anspruch 4, dadurch gekennzeichnet, daß

- an der beweglichen Backe (24) innerhalb des äußeren Rohres (21) ein im Querschnitt V-förmiges, mit seiner Scheitelkante in Richtung auf die feststehende Backe (23) weisendes Teil (61) angebracht ist,
- die Scheitelkante senkrecht zur Achse des äußeren Rohres (21) verläuft und in einer senkrecht zur Achse des äußeren Rohres (21) verlaufenden Ausnehmung (46) gelagert ist,
- am distalen Ende des äußeren Rohres (21) ein Stift (35) derart angebracht ist, daß durch proximales Verschieben des äußeren Rohres (21) relativ zu dem Instrumentenschaft (20) das im Querschnitt V-förmigen Teil durch den Stift (35) in der Ausnehmung (46) gedreht wird, wodurch sich die bewegliche Backe (24) von der feststehenden Backe (23) entfernt.

10. Chirurgisches Nähinstrument nach Anspruch 4, dadurch gekennzeichnet, daß die bewegliche Backe (24) an ihrer der feststehenden Backe (23) abgewandten Seite eine Aufwölbung (37) aufweist, durch die sie bei distal bewegtem äußerem Rohr (21) über die parallele Stellung hinaus eine Bewegung in Richtung auf die feststehende Backe (23) ausführt.

11. Chirurgisches Nähinstrument nach einem der Ansprüche 1 bis 8, dadurch gekennzeichnet, daß eine der beiden Backen (23, 24) auf der der anderen Backe gegenüberliegenden Seite (36, 40) einen Dorn aufweist, der in der parallelen Stellung in eine Vertiefung in der anderen Backe eingreift.

## Claims

1. Surgical suturing instrument,

   a) having an instrument shaft (20), which includes a distal end, which is to be inserted into the body of a patient, and a proximal end,
   b) having two oppositely situated jaws (23, 24) at the distal end of the instrument shaft (20),
   c) the first jaw (23) being securely connected to the instrument shaft (20), and
   d) the second jaw (24) being displaceably disposed on the instrument shaft (20) opposite the first jaw (23),
   e) having a channel (43) in the instrument shaft (20) for supplying a suturing material (17) from the proximal end to the distal end, which channel terminates on that side (40) of the first, stationary jaw (23) lying opposite the second, displaceable jaw (24),
   f) having an outer tube (21), which is axially displaceable relative to the instrument shaft (20) and is disposed between the distal end and the proximal end of the instrument shaft (20), such tube surrounding said shaft, but leaving the jaws (23, 24) at least partially free,
   g) having a multi-part handle (1, 2) at the proximal end of the instrument shaft (20) to actuate the displaceable jaw, one part (2) of said handle being connected to the proximal end of the instrument shaft (20) and an additional part (1) being connected to the outer tube (21),

   characterised in that

   h) the two jaws (23, 24) can assume a positon relative to each other in which they are disposed approximately parallel to each other with a spacing therebetween over their entire length, and
   i) a plurality of grooves (38) are provided both in the displaceable jaw (24) and in the stationary jaw (23) in their oppositely situated sides (36, 40) extending from the channel (43) for the supply of the suturing material (17), said grooves being disposed with a spacing therebetween and the bottom surfaces thereof lying on a helical line in this position.

2. Surgical suturing instrument according to claim 1, characterised by means (14, 28), whereby the suturing material (17) is stepwisely distally displaceable through the channel (43) via the handle (1, 2) in such a manner that it passes from the end of the channel initially into the first of the grooves (38) of the displaceable jaw (24), then into the first of the grooves (38) of the stationary jaw (23), and finally into the additional grooves of the displaceable jaw (24) and of the stationary jaw (23) alternately.

3. Surgical suturing instrument according to claim 2, characterised by a blocking device (57, 58), which acts upon the suturing material (17) in the channel (43), and which releases the suturing material (17) only during distal displacement.

4. Surgical suturing instrument according to claim 1 or 2, characterised in that the displaceable jaw (24) is displaceable from the parallel position both towards and away from the stationary jaw (23) via the handle (1, 2).

5. Surgical suturing instrument according to claim 1 or 4, characterised in that the outer tube (21) has, at its distal end, two oppositely situated notches (25), which are disposed symmetrically relative to a plane extending parallel to the side (40) of the stationary jaw (23), which lies opposite the displaceable jaw (24).

6. Surgical suturing instrument according to claim 1, characterised in that the two jaws (23, 24) have, on their oppositely situated sides (36, 40) in the distal region before the grooves (38), closely adjacent indentations (62) which extend parallel to the grooves (38), the spacing between said indentations being 1/n of the spacing between the grooves (38), n being a whole number.

7. Surgical suturing instrument according to claim 1 or 2, characterised in that the grooves (38) are widened in the form of a funnel (50) at the ends which are initially reached by the suturing material (17) during its distal displacement.

8. Surgical suturing instrument according to one of claims 1 to 7, characterised in that the stationary jaw (23) includes a displaceable carriage (45) provided with a cutting edge (41), by means of which the suturing material (17) can be severed.

9. Surgical suturing instrument according to claim 4, characterised in that

   - a part (61), which has a V-shaped cross-section and is orientated towards the stationary jaw (23) with its vertical edge, is attached to the displaceable jaw (24) internally of the outer tube (21),
   - the vertical edge extends perpendicularly relative to the axis of the outer tube (21) and is mounted in a recess (46), which extends perpendicularly relative to the axis of the outer tube (21),
   - a pin (35) is attached to the distal end of the outer tube (21) so that the portion with the V-shaped cross-section is rotated by the pin (35) in the recess (46) as a result of the proximal displacement of the outer tube (21) relative to the instrument shaft (20), whereby the displaceable jaw (24) moves away from the stationary jaw (23).

10. Surgical suturing instrument according to claim 4, characterised in that the displaceable jaw (24) has, on its side remote from the stationary jaw (23), an arched portion (37) by means of which said displaceable jaw executes a movement towards the stationary jaw (23) when the outer tube (21) is moved distally beyond the parallel position.

11. Surgical suturing instrument according to one of claims 1 to 8, characterised in that one of the two jaws (23, 24) on the side (36, 40) situated opposite the other jaw, has a mandrel which engages in a recess in the other jaw in the parallel position.

**Revendications**

1. Instrument chirurgical de suture :

   a) avec une tige d'instrument (20), qui comporte une extrémité distale, à introduire dans le corps d'un patient et une extrémité proximale,
   b) avec deux mâchoires (23, 24) opposées l'une à l'autre à l'extrémité distale de la tige de l'instrument (20), tandis que,
   c) la première mâchoire (23) est reliée de manière fixe à la tige de l'instrument (20) et,
   d) la deuxième mâchoire (24) est disposée mobile par rapport à la première mâchoire (23) sur la tige de l'instrument (20),
   e) avec un canal (43) dans la tige de l'instrument (20) pour amener un produit de suture (17) de l'extrémité proximale à l'extrémité distale, canal qui se termine sur ce côté (40) de la première mâchoire fixe (23), qui fait face à la deuxième mâchoire mobile (24),
   f) avec un tube extérieur (21) pouvant coulisser axialement par rapport à la tige de l'instrument (20), qui est placé entre l'extrémité distale et l'extrémité proximale de la tige d'instrument (20), entoure celle-ci, mais laisse libre au moins partiellement les mâchoires (23, 24),
   g) avec une manette en plusieurs pièces (1, 2) à l'extrémité proximale de la tige d'instrument (20) pour commander la mâchoire mobile, dont une partie (2) est reliée à l'extrémité proximale de la tige de l'instrument (20) et une autre partie (1) au tube extérieur (21),

   caractérisé en ce que

   h) les deux mâchoires (23, 24) peuvent prendre une position l'une par rapport à l'autre, dans laquelle elles sont disposées sur leur longueur totale à une distance approximativement parallèle l'une par rapport à l'autre,
   i) dans leurs faces (36, 40) opposées l'une à l'autre partant du canal (43) pour l'alimentation de la matière de suture (17) aussi bien dans la mâchoire mobile (24) que dans la mâchoire fixe (23) sont mises en oeuvre plusieurs rainures (38) disposées à une certaine distance l'une par rapport à l'autre, dont les surfaces de base sont situées dans cette position sur une hélice.

2. Instrument de chirurgical de suture selon la revendication 1,
   caractérisé par
   des moyens (14, 28) à l'aide desquels le produit de suture (17) peut coulisser distalement pas à pas à travers le canal (43) par l'intermédiaire des pièces de prise (1, 2) de manière, qu'il arrive de l'extrémité du canal, d'abord dans la première des rainures (38) de la mâchoire mobile (24), ensuite dans la première des rainures de la mâchoire fixe (23) et enfin alternativement dans les autres rainures de la mâchoire mobile (24) et de la mâchoire fixe (23).

3. Instrument chirurgical de suture selon la revendication 2,
   caractérisé par
   un dispositif de découpe (57, 58) qui agit sur la matière de suture (17) dans le canal (43), et qui libère la matière de suture (17) seulement lors du coulissement distal.

4. Instrument chirurgical de suture selon la revendication 1 ou 2,
   caractérisé en ce que
   la mâchoire mobile (24) peut être déplacée par l'intermédiaire des pièces de prise (1, 2) en partant de la position parallèle aussi bien vers la mâchoire fixe (23) qu'en s'éloignant d'elle.

5. Instrument chirurgical de suture selon la revendication 1 ou 4,
   caractérisé en ce que
   le tube extérieur (21) comporte à son extrémité distale deux entailles (25) opposées l'une à l'autre, qui sont disposées symétriquement par rapport à un plan, qui se développe parallèlement au côté (40) de la mâchoire fixe (23), qui se situe en face de la mâchoire mobile (24).

6. Instrument chirurgical de suture selon la revendication 1,
   caractérisé en ce que
   les deux mâchoires (23, 24) comportent sur leurs faces (36, 40) opposées dans la zone distale avant les rainures (38) des enfoncements (62) étroitement voisines qui se développent parallèlement aux rainures (38) et dont l'écartement de l'une à l'autre se monte à 1/n de la distance des rainures (38), n représentant un nombre entier.

7.  Instrument chirurgical de suture selon la revendication 1 ou 2,
    caractérisé en ce que
    les rainures (38) sur les extrémités, qui sont d'abord atteintes par le produit de suture (17) lors de son déplacement distal, sont élargies à la manière d'un entonnoir (50).

8.  Instrument chirurgical de suture selon une des revendications 1 à 7,
    caractérisé en ce que
    la mâchoire fixe (23) contient un poussoir mobile (45) avec une arête de coupe (41), avec laquelle le produit de suture (17) peut être détaché.

9.  Instrument chirurgical de suture selon la revendication 4,
    caractérisé en ce que

    -   sur la mâchoire mobile (24) à l'intérieur du tube extérieur (21) est montée une pièce (61) de section transversale en forme de V, comportant son arête au sommet en direction de la mâchoire fixe (23),
    -   l'arête au sommet se développe perpendiculairement à l'axe du tube extérieur (21) et est logée dans un évidement (46) se développant perpendiculairement à l'axe du tube extérieur (21),
    -   à l'extrémité distale du tube extérieur (21) est appliquée une cheville (35), de façon que par un déplacement proximal du tube extérieur (21) par rapport à la tige de l'instrument (20), la pièce de section transversale en forme de V par la cheville (35) tourne dans l'évidement (46), grâce à quoi la mâchoire mobile (24) s'éloigne de la mâchoire fixe (23).

10. Instrument chirurgical de suture selon la revendication 4,
    caractérisé en ce que
    la mâchoire mobile (24) comporte sur sa face éloignée de la mâchoire fixe (23) une formation en voûte (37), par laquelle elle effectue dans le cas du tube (21) extérieur déplacé distalement au-delà de la position parallèle, un mouvement en direction de la mâchoire fixe (23).

11. Instrument chirurgical de suture selon une des revendications 1 à 8,
    caractérisé en ce qu'
    une des deux mâchoires (23, 24) comporte sur le côté (36, 40) opposé à l'autre mâchoire une broche, qui s'insère dans la position parallèle dans une cavité de l'autre mâchoire.

Fig. 1

Fig. 2

Fig. 3a

EP 0 641 178 B1

Fig. 3b

EP 0 641 178 B1

Fig. 4

Fig. 5

Schnitt A-A  Schnitt C-C  Schnitt E-E

Fig. 6

EP 0 641 178 B1

Fig. 7